# DEMANDE DE BREVET EUROPEEN

(11) **EP 4 497 938 A1**
(43) Date de publication de la demande: **29.01.2025**
(21) Numéro de dépôt: 24191007.4
(22) Date de dépôt: 25.07.2024
(51) Int. Cl.: F02K 9/08, F02K 9/38, F02K 9/96, G01N 3/32, G01N 11/16

(54) **PROPULSEUR À PROPERGOL SOLIDE À MOYEN DE CONTRÔLE INTÉGRÉ ET SYSTÈME DE CONTRÔLE DE VIEILLISSEMENT DU PROPULSEUR**

(30) Priorité: 27.07.2023 FR 2308136
(71) Demandeur: ROXEL France, 33160 Saint Médard en Jalles (FR)
(72) Inventeur: ALARY, Claude, 33160 Saint-Médard-en-Jalles (FR)
(74) Mandataire: Brevalex

(57) **Abrégé**

L'invention concerne un propulseur à propergol solide comprenant une enveloppe (12) abritant un chargement de propergol (14), un canal (16) axial étant réalisé dans le chargement de propergol (14) et permettant la combustion du chargement de propergol (14), l'enveloppe (12) portant une éprouvette (24) de propergol disposée dans le canal (16) et un transducteur (28) disposé à l'extérieur de l'enveloppe (12) et en regard de l'éprouvette (24) de façon à permettre l'émission d'une sollicitation vibratoire au travers de l'éprouvette (24) et d'en transmettre une réponse.

## Description

L'invention concerne un propulseur à propergol solide à moyen de contrôle intégré et un système de contrôle de vieillissement de propulseur.

De nombreux engins balistiques de type fusée mettent en oeuvre la combustion d'un propergol solide pour délivrer un effort de poussée assurant leur déplacement.

Les engins balistiques, après leur fabrication, peuvent être stockés durant des périodes parfois très longues pouvant atteindre plusieurs années. Il est important de connaître l'état du propergol avant son utilisation afin de vérifier s'il est apte à remplir sa fonction, pour évaluer le niveau de sécurité inhérente à son exploitation.

Il est possible de définir l'âge apparent du propergol correspondant à un niveau de vieillissement effectivement constaté. Cela permet d'évaluer la durée de vie encore disponible pour l'engin balistique mettant en oeuvre le propergol.

Les propergols solides contiennent à la fois un combustible et un comburant et la connaissance de certaines caractéristiques mécaniques du propergol, comme le module d'Young ou le module de cisaillement, permet d'en estimer l'âge apparent. Ces caractéristiques mécaniques peuvent être retrouvées à partir de la réponse du matériau à la sollicitation d'une onde vibratoire.

Il est connu, notamment du document FR 2 954 498 A1, de mettre en oeuvre un dispositif de test contenant, dans un boitier, un échantillon de propergol et un système vibratoire. Le dispositif de test peut être placé à proximité de l'engin balistique voire même à l'intérieur de celui-ci. Afin de tenter d'obtenir un vieillissement comparable pour l'engin balistique et pour le dispositif de test.

Cependant, le dispositif de test décrit dans le document FR 2 954 498 A1 n'est pas adapté à une exploitation industrielle. Plusieurs inconvénients ont été recensés par la demanderesse :
- Le dispositif est lourd et volumineux à l'intérieur du propulseur.
- Les conditions d'environnement de l'échantillon de propergol du dispositif de test ne sont pas le mêmes que celles du propergol du propulseur du fait du confinement de l'échantillon à l'intérieur du boitier du dispositif de test. Notamment, l'oxydation de l'échantillon ne se fait pas à la même vitesse que celle du propergol du propulseur.
- La mise en place du dispositif de test à l'intérieur du propulseur entraine la présence d'une source d'énergie électrique à l'intérieur du propulseur. Ceci présente un danger d'allumage du propulseur durant une phase de test de l'échantillon.
- La présence de composants autres que du propergol à l'intérieur du propulseur peut en perturber le fonctionnement.

L'invention vise à pallier tout ou partie des problèmes cités plus haut en proposant un propulseur à propergol solide équipé d'une éprouvette de test permettant, de l'extérieur du propulseur, d'émettre une sollicitation vibratoire au travers de l'éprouvette et d'en transmettre une réponse.

A cet effet, l'invention a pour objet un propulseur à propergol solide comprenant une enveloppe abritant un chargement de propergol, un canal axial étant réalisé dans le chargement de propergol et permettant la combustion du chargement de propergol, l'enveloppe portant une éprouvette de propergol disposée dans le canal et un transducteur disposé à l'extérieur de l'enveloppe et en regard de l'éprouvette de façon à permettre l'émission d'une sollicitation vibratoire au travers de l'éprouvette et d'en transmettre une réponse.

Avantageusement, le canal s'étend entre deux extrémités axiales du propulseur, l'enveloppe présentant un orifice de passage de l'éprouvette communiquant avec le canal à l'une de ses extrémités.

Avantageusement, le propulseur comporte en outre un disque fixé à l'enveloppe au niveau de l'orifice et présentant une face interne orientée vers le canal et sur laquelle est fixée l'éprouvette ainsi qu'une face externe orientée à l'opposé du canal et sur laquelle est fixée le transducteur.

Avantageusement, l'enveloppe présente un renfoncement cylindrique dans lequel est logé le disque, l'orifice de passage de l'éprouvette étant ménagé dans le fond du renfoncement cylindrique.

Avantageusement, l'éprouvette est fixée sur une face interne du disque au moyen d'une colle.

Le chargement de propergol peut comprendre un liant polymère et la colle est avantageusement à base du même liant polymère.

Avantageusement, le propergol de l'éprouvette est identique de celui du chargement.

Avantageusement, le transducteur comprend des connexions électriques permettant de recevoir un premier signal configuré pour que le transducteur émette la sollicitation vibratoire et permettant de récupérer un second signal représentatif de la réponse de l'éprouvette à la sollicitation vibratoire.

L'invention a également pour objet un système de contrôle de vieillissement d'un propulseur selon l'invention, comprenant un module électronique configuré pour :
- se raccorder temporairement au transducteur,
- émettre un premier signal vers le transducteur lui permettant de former la sollicitation vibratoire,
- recevoir un second signal de réponse du transducteur,
- déduire à partir du signal reçu un paramètre représentatif d'un état de vieillissement du chargement du propulseur.

L'invention sera mieux comprise et d'autres avantages apparaîtront à la lecture de la description détaillée d'un mode de réalisation donné à titre d'exemple, description illustrée par le dessin joint dans lequel :

la [Fig. 1] représente schématiquement un propulseur à propergol solide selon l'invention ;

la [Fig. 2] représente plus en détail des composants permettant de tester le propergol du propulseur de la figure 1.

Par souci de clarté, les mêmes éléments porteront les mêmes repères dans les différentes figures.

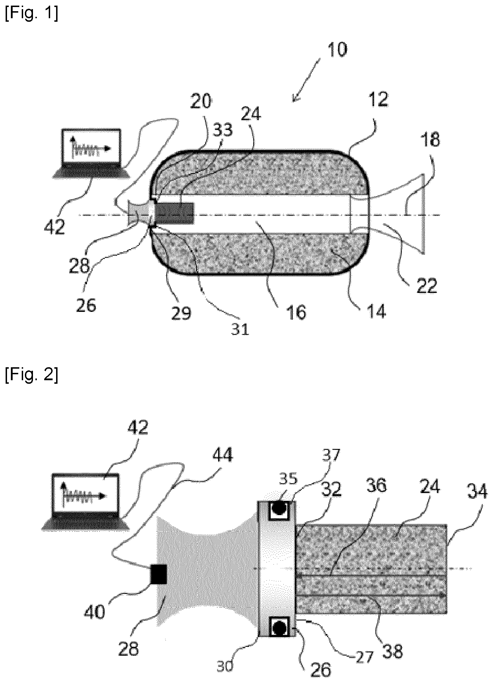

Les figures 1 et 2 représente schématiquement un propulseur 10 par exemple mis en oeuvre dans un engin balistique. Le propulseur 10 comprend à l'intérieur d'une enveloppe 12 un chargement de propergol solide 14. Le propergol solide contient à la fois un carburant et un comburant. Le propergol est par exemple un propergol composite sur base de liant polyuréthane tel que décrit dans le brevet français FR 3 108 331 B3 déposé au nom de la demanderesse. L'invention n'est pas limitée à ce type de propergol et tout type de propergol peut être mis en oeuvre dans le cadre de l'invention.

La combustion du propergol assure la poussée du propulseur et l'enveloppe 12 doit résister à la pression et à la température due à la combustion. Pour résister à la température de combustion, il est possible d'intégrer une protection thermique entre l'enveloppe 12 et le chargement de propergol. Pour résister à la pression différents matériaux peuvent être mis en oeuvre comme par exemple un matériau métallique tel qu'un acier ou un matériau à base de fibres de carbone. D'autres matériaux peuvent être mis en oeuvre et d'autres contraintes que la pression et la température pourront apparaitre plus loin pour la mise en oeuvre de l'invention.

Un canal longitudinal 16, s'étendant selon un axe 18, est réalisé dans le chargement de propergol 14. Le canal 16 s'étend entre deux extrémités axiales 20 et 22 du propulseur 10. L'une des extrémités 20 du canal 16 est ici obstruée par un disque 26 et l'autre extrémité 22 du canal 16 forme une tuyère par laquelle sont éjectés les produits de combustion du propergol assurant la propulsion.

Selon l'invention, le propulseur 10 comprend une éprouvette 24 de propergol disposée dans le canal 16 et fixée ici sur une face interne 27 du disque 26. L'éprouvette 24 de propergol est avantageusement réalisée dans le même propergol que celui du chargement de propergol 14. Alternativement, il est possible de mettre en oeuvre pour l'éprouvette 24 un propergol différent de celui du chargement 14 mais ayant des propriétés physiques voisines, notamment en terme de vieillissement.

Le propulseur 10 comprend en outre un transducteur 28 fixé sur une face externe 30 du disque 26 et disposé en regard de l'éprouvette 24 de façon à permettre l'émission d'une sollicitation vibratoire au travers de l'éprouvette 24 et d'en transmettre une réponse. Le canal 16 peut être directement réalisé dans le chargement de propergol 14. Dans ce cas, l'éprouvette 24 est avantageusement nue. Plus précisément, le propergol de l'éprouvette 24 ainsi que celui du chargement 14 sont directement exposés à l'air se trouvant dans le canal 16. Ainsi le propergol du chargement 14 et celui de l'éprouvette 24 réagissent de la même façon à l'environnement du canal 16. Plus précisément, lors du stockage du propulseur 10, des variations de température et d'humidité peuvent intervenir. Ces deux paramètres sont déterminants dans le vieillissement du propergol. D'autres paramètres peuvent également intervenir comme par exemple la pression mais sont a priori moins déterminants. Cependant le fait d'exposer le propergol du chargement 14 et celui de l'éprouvette 24 au même environnement permet de prendre en compte tout type de paramètre d'environnement. Cette exposition identique permet en outre de s'adapter à des compositions différentes de propergol pouvant vieillir différemment en fonction de l'environnement ; en conservant un même matériau pour le propergol du chargement 14 et celui de l'éprouvette 24, ceux-ci vieilliront de façon semblable car exposés aux mêmes conditions d'environnement.

La figure 2 représente plus en détail le transducteur 28, le disque 26 et l'éprouvette 24. Le transducteur 28 est disposé sur la face externe 30 du disque 26 portant l'éprouvette 24 sur sa face interne 27, ici parallèle à la face externe 30, et est configuré pour émettre une sollicitation vibratoire dans l'éprouvette 24. La sollicitation vibratoire est par exemple obtenue au moyen d'une onde de type acoustique ultrasonore émise par le transducteur 28 et traversant le disque 26 et l'éprouvette 24. Un disque 26 en acier possède notamment de bonnes propriétés de transmission d'une sollicitation vibratoire de type ultrasonore. Plus généralement, le matériau de l'insert que forme le disque 26 est choisi pour qu'il puisse transmettre une sollicitation mécanique en étant peu soumis au risque de vieillissement comme le propergol.

L'éprouvette 24 possède, dans l'exemple représenté sur les figures 1 et 2 deux faces parallèles, l'une 32 en contact avec la face interne 27 du disque 26 et l'autre 34 libre et exposée à l'air présent dans le canal 16. La sollicitation vibratoire émise par le transducteur 28 traverse une première fois l'éprouvette 24 depuis sa face 32 jusqu'à sa face 34, se reflète au moins partiellement sur la face 34, traverse à nouveau le disque 26 pour être reçue par le transducteur 28. Le trajet de la sollicitation vibratoire, dit trajet aller, depuis la face 32 vers la face 34 est matérialisé par une flèche 36 et le trajet de la sollicitation vibratoire, dit trajet retour, depuis la face 34 vers la face 32 est matérialisé par une flèche 38. D'autres formes d'éprouvette 24 sont possibles pour mettre en oeuvre l'invention. Les faces 32 et 34 peuvent être non parallèles. Les faces 32 et 34 ne sont pas obligatoirement planes. Par exemple, la face interne 27 du disque 26 sur laquelle est disposée l'éprouvette 24 peut ne pas être plane et il est avantageux que la face 32 de l'éprouvette 24 se conforme à la forme de la face interne 27 afin de favoriser la transmission de la sollicitation vibratoire directement du disque 26 vers l'éprouvette 24 sans passer par une éventuelle lame d'air pouvant se trouver entre la face interne 27du disque 12 et la face 32 de l'éprouvette 24.

L'épaisseur du disque 12 lui permet de supporter le couple température/ pression régnant dans la chambre de combustion mais, en tant que de besoin, une feuille de protection thermique peut être intercalée entre ces deux faces 27 et 32 pour protéger thermiquement le disque 26, sans que cela perturbe fondamentalement le principe acoustique du système.

Avantageusement, l'éprouvette 24 est collée sur la face interne 27 du disque 26 tandis que le transducteur 28 est collé sur la face externe 30 du disque 26. Ainsi aucun composant mécanique susceptible de perturber la sollicitation vibratoire n'est présent dans les fixations de l'éprouvette 24 et du transducteur 28. Le collage permet également de limiter la formation d'une lame d'air entre la face interne 27 du disque 26 et la face 32 de l'éprouvette 24 en se diffusant complètement entre les faces 27 et 32. Il en va de même du côté du transducteur 28. Le propergol peut comprendre un liant polymère, comme par exemple décrit dans le brevet FR 3 108 331 B3. Il est alors avantageux de mettre en oeuvre une colle 39 à base du même liant que celui mis en oeuvre dans le propergol pour fixer l'éprouvette 24 sur la face interne 27 du disque 26, voire le transducteur 28 sur sa face opposée 30. Ce type de colle permet notamment de moins perturber la propagation de la sollicitation vibratoire à l'interface entre la colle et le propergol.

En variante, un collage de de l'éprouvette 24 directement sur la face interne de l'enveloppe 12 est envisageable avec une enveloppe obstruant l'extrémité 20 du canal 16.

La fixation du disque 26 à l'enveloppe peut être réalisée de différentes façons. Il est ici logé dans un renfoncement cylindrique 29 de l'enveloppe 12 et fixé à celui-ci par vissage (non représenté sur les figures dans un souci de simplification). Le fond 31 du renfoncement 29 présente un orifice 33 pour laisser passer l'éprouvette 24. Il est par exemple aussi possible, en variante, de brider mécaniquement le disque 26 sur la face externe de l'enveloppe 12.

Un joint d'étanchéité 35 est par ailleurs interposé entre le disque 26 et l'enveloppe 12, en étant ici logé dans une gorge 37 ménagée dans le disque 26.

Sur les figures 1 et 2, le transducteur 28 est représenté comme un composant monobloc configuré pour émettre une sollicitation vibratoire et pour recevoir une réponse à la sollicitation vibratoire émise. En pratique, il est possible de distinguer les deux fonctions : émission et réception de la sollicitation vibratoire, un premier composant pour émettre la sollicitation et un second pour recevoir la sollicitation vibratoire réfléchie par la face 34 après avoir traversée l'éprouvette 24. Le transducteur 28 peut générer la sollicitation vibratoire au moyen d'une céramique piézo-électrique. Ce type de céramique permet également de recevoir l'onde réfléchie, formant la réponse à la sollicitation vibratoire, et en délivrer un signal électrique correspondant.

Dans le mode de réalisation représenté, le transducteur 28 comprend des connexions électriques 40 permettant de raccorder temporairement le transducteur 28. Les connexions électriques 40 et le transducteur 28 sont à l'extérieur de l'enveloppe 12, ce qui permet d'éviter que tout signal électrique ne pénètre dans l'enveloppe pour faire fonctionner le transducteur 28. Ainsi ne pénètre dans l'enveloppe 12 que la sollicitation vibratoire. Les connexions électriques 40 permettent de raccorder un module électronique 42 représenté ici sous forme d'un ordinateur personnel. Le module électronique 42 peut être raccordé au transducteur 28 au moyen d'un câble 44. Alternativement le module électronique 42 peut être raccordé au transducteur 28 au moyen d'une liaison sans fil.

Le module électronique 42 est configuré pour générer et émettre un signal vers le transducteur 28 lui permettant de former la sollicitation vibratoire. Le module électronique 42 est également configuré pour recevoir un signal de réponse du transducteur 28, signal correspondant à la réponse vibratoire de l'éprouvette 24.

Le module électronique 42 est configuré pour analyser le signal de réponse du transducteur 28 et en déduire un paramètre représentatif de l'état de vieillissement du propergol de l'éprouvette 24 et donc de l'état de vieillissement du propergol du chargement 14. Cette déduction se fait par exemple en comparant le signal reçu avec des signaux mémorisés représentatifs de différents états de vieillissement. Il est également possible de déterminer certains paramètres mécaniques du propergol comme son module d'Young ou son module de cisaillement à partir de la réponse vibratoire. Les évolutions de ces paramètres mécaniques renseignent sur l'état de vieillissement du propergol.

On observera à cet égard que les dispositions selon l'invention permettent de déterminer la durée de vie résiduelle en service d'un propulseur sans avoir notamment besoin de connaître les conditions dans lesquelles le propulseur a été stocké pour déterminer l'état vieillissement du propergol.

## Revendications

1. Propulseur à propergol solide comprenant une enveloppe (12) abritant un chargement de propergol (14), un canal (16) axial étant réalisé dans le chargement de propergol (14) et permettant la combustion du chargement de propergol (14), l'enveloppe (12) portant une éprouvette (24) de propergol disposée dans le canal (16) et un transducteur (28) disposé à l'extérieur de l'enveloppe (12) et en regard de l'éprouvette (24) de façon à permettre l'émission d'une sollicitation vibratoire au travers de l'éprouvette (24) et d'en transmettre une réponse.

2. Propulseur selon la revendication 1, dans lequel le canal (16) s'étend entre deux extrémités (20, 22) axiales du propulseur (10), l'enveloppe (12) présentant un orifice (33) de passage de l'éprouvette (24) communiquant avec le canal (16) à l'une de ses extrémités (20).

3. Propulseur selon la revendication 2, comportant en outre un disque (26) fixé à l'enveloppe (12) au niveau de l'orifice (33) et présentant une face interne (27) orientée vers le canal (16) et sur laquelle est fixée l'éprouvette (24) ainsi qu'une face externe (30) orientée à l'opposé du canal (16) et sur laquelle est fixée le transducteur (28).

4. Propulseur selon la revendication 3, dans lequel l'enveloppe (12) présente un renfoncement cylindrique (29) dans lequel est logé le disque (26), l'orifice (33) de passage de l'éprouvette étant ménagé dans le fond (31) du renfoncement cylindrique (29).

5. Propulseur selon l'une des revendications 3 et 4, dans lequel l'éprouvette (24) est fixée sur une face interne (27) du disque (26) au moyen d'une colle (39).

6. Propulseur selon la revendication 5, dans lequel le chargement de propergol (14) comprend un liant polymère et dans lequel la colle (39) est à base du même liant polymère.

7. Propulseur selon l'une des revendications précédentes, dans lequel le propergol de l'éprouvette (24) est identique de celui du chargement (14).

8. Propulseur selon l'une des revendications précédentes, dans lequel le transducteur (28) comprend des connexions électriques (42) permettant de recevoir un premier signal configuré pour que le transducteur (28) émette la sollicitation vibratoire et permettant de récupérer un second signal représentatif de la réponse de l'éprouvette (24) à la sollicitation vibratoire.

9. Système de contrôle de vieillissement d'un propulseur (10) selon l'une des revendications précédentes, comprenant un module électronique (42) configuré pour :
- se raccorder temporairement au transducteur (28),
- émettre un premier signal vers le transducteur (28) lui permettant de former la sollicitation vibratoire,
- recevoir un second signal de réponse du transducteur (28),
- déduire à partir du signal reçu un paramètre représentatif d'un état de vieillissement du chargement (14) du propulseur (10).
